# EUROPEAN PATENT SPECIFICATION

(11) **EP 1 677 777 B1**
(45) Date of publication and mention of the grant of the patent: **18.04.2007**
(21) Application number: 04791654.9
(22) Date of filing: 08.10.2004
(51) Int. Cl.: A61K 31/137, A61K 9/16, A61K 47/02, A61K 47/38

(54) **PELLETS CONTAINING VENLAFAXINE HYDROCHLORIDE**
PELLETS MIT VENLAFAXIN-HYDROCHLORID
GRANULES CONTENANT DU CHLORHYDRATE DE VENLAFAXINE

(30) Priority: 10.10.2003 HU 0303382
(43) Date of publication of application: 12.07.2006
(73) Proprietor: EGIS Gyógyszergyár Nyrt, 1106 Budapest (HU)
(72) Inventor: FEKETE, Pál, H-1141 Budapest (HU); KORBÉLY, Tibor, H-2484 Agárd (HU); BOZSO, Agnes, H-2060 Bicske (HU); MORICZ, Eszter, H-1157 Budapest (HU)
(74) Representative: Beszédes, Stephan G.
(86) International application number: PCT/HU2004/000095
(87) International publication number: WO 2005/034930

(56) References cited:
- EP-A- 1 331 003
- WO-A1-03/041692
- WO-A1-03/082261

## Description

### Field of the invention

The invention relates to pellets containing venlafaxine hydrochloride and a process for the preparation thereof.

### Technical background of the invention

Venlafaxine (1-[2-(dimethylamino)-1-(4-methoxyphenyl)ethyl]cyclohexanol) is a highly important antidepressant belonging to the antipsychotics possessing serotonin/noradrenalin uptake inhibiting effect (SNRI). A drawback of immediate release pharmaceuticals in therapeutic application resides in the fact that the quick release of the active agent results in a high plasma concentration, which causes unpleasant side-effects, such as nausea or vomiting in a considerable part of the patients. In order to avoid high plasma concentration following administration in case of venlafaxine as active ingredient the administration of sustained release pharmaceutical dosage forms is very advantageous, because the slow release of the active ingredient results in a lower and even blood level and thus the side-effects can be decreased.

The most advantageous forms of sustained release pharmaceutical dosage forms are pharmaceuticals containing drug-loaded pellets. Pellets are spherical agglomerates 0,2-2 mm in diameter, which may be coated with one or more layer(s) regulating the release of the active agent and filled into hard gelatine capsules or tabletted When administering these compositions to patients, in the stomach the capsules or tablets are disintegrated or distributed into individual pellets, which are then thoroughly mixed with the content of the stomach, and the discharge of the pellets from the stomach becomes more uniform. In this way a high active agent concentration cannot develop during the release of the active ingredient from the pellets, thus the plasma concentration becomes more even. The therapeutic activity of the composition becomes more favourable and the likelihood of the occurrence of side-effects considerably decreases.

The first pellet-based pharmaceutical compositions were put on the market in the early 1950s. However, they could not become more widespread until the 1970s due to their cumbersome preparation technology, which was based on the layering of the active ingredient and solid auxiliary agents, such as various types of starch, onto sugar crystals in coating drums, and in this way the pellet preparation took several days.

From the beginning of the 1970s a special equipment is available for the preparation of pellets. This equipment serves basically for the purpose of extrusion-spheroidization, centrifugal granulation, fluidization rotogranulation and high-shear mixing processes. By applying said equipment pellets can be produced directly, within only a few hours from a suitable powder mixture. Recently especially the application of fluidization rotary granulators and high-shear pelletizing machines combined with vacuo or microwave drying has become frequent, because by using such an equipment the production of pellets can be performed in a single apparatus (Isaac Ghebre-Sellassie: Pharmaceutical Pelletization Technology, Marcel Dekker, Inc., New York, Basel, 1989).

Pellets can be prepared according to a number of methods, which all can be based on the following tree principles:
1. layering
2. extrusion-spheroidization
3. build-up granulation

By the layering method a mixture of the particles of the active ingredient and/or the auxiliary agents of small particle size is applied in layers onto a nearly isodiametric seed material having larger particle size than that of the said active ingredient and/or auxiliary agents. The application can be performed in a conventional coating drum, in a centrifugal granulating equipment or in a fluidization spraying apparatus. In certain cases in the fluidization machines the active ingredient can be sprayed onto the surface of the inert granules from a solution. However, according to Hungarian patent specification No. 199,677 the high solubility (exceeding 600 mg/ml) of the active ingredient is disadvantageous from the viewpoint of the layering pelletization.

In case of the extrusion-spheroidization process the active ingredient is mixed with the auxiliary agents and a liquid, the wet mass is fed into an extruder having holes of about 1 mm in diameter, and the extrudate is formed into uniform, nearly spherical particles in a rotary spheroidization machine.

According to the build-up granulation process a mixture of the active ingredient and the auxiliary agents is mixed in a mixer or in a centrifugal or fluidization granulator, while a liquid is fed into the machine. During this process the particles of the powder mixture adhere to each other and become spherical upon the effect of shear forces and abrasive forces.

The last step of all pelletization processes is drying of the particles and separation of the fraction having a particle size suitable for further processing. The quality of the pelletization technology can be characterized by the product fraction, that is by the ratio of the mass of the pellets having the desired particle size to the total mass of the pellets. The pellets of unsuitable size are namely recycled to the manufacturing process and processed repeatedly, which results in an increase of the production time, costs of material and energy and the intensity of the labour involved in the procedure.

In case of pellets filled into capsules the preferable particle size varies between 0.5 mm and 1.0 mm, in case of pellets compressed into tablets between 0.3 mm and 0.6 mm. In case of active ingredients readily soluble in water it is expedient to use pellets of bigger particle size, such as 0. 8 - 1.6 mm or 1 . 0 - 2.0 mm, in order to decrease the amount of the coating material necessary for the coating modifying the release of the active ingredient.

In addition to the active ingredient pharmaceutically acceptable excipients are also applied for the pelletization, which promote the formation of pellets of appropriate shape and surface. Such excipients may be fillers, lubricants, antiadhesives, disintegrants or drug release promoting additives, buffers, surfactants, surface-active substances, pelletization promoting additives or glidants (Isaac Ghebre-Sellassie: Pharmaceutical Pelletization Technology, Marcel Dekker, Inc., New York, Basel, 1989).

As fillers which can be used for the pelletization e.g. calcium sulfate, calcium hydrophosphate, lactose, mannitol, szaccharose, starch and microcrystalline cellulose are mentioned. Suitable binders are e.g. gelatine, hydroxy-propyl cellulose, hydroxypropyl-methyl cellulose, methylcellulose, polyvinyl pyrrolidone, szaccharose and starch. As lubricant calcium stearate, hydrogenated vegetable oil, magnesium stearate, mineral oil, polyethylene glycol, glycerine and propylene glycol may be applied.

The pellets may also contain antiadhesives, such as kaolin, talk or silicon dioxide, furthermore disintegrants or drug release promoting agents, e.g. alginates, sodium carboxymethyl cellulose, crospovidon, starch, pre-gelatinized starch, sodium carboxymethyl starch, or e.g. ethylcellulose, carnauba vax, shellac. Buffers (such as citrate, phosphate, carbonate and hydrocarbonate salts), surface-active additives (such as polysorbate, sodium laurylsulfate) and spheroidization promoting substances (e.g. microcrystalline cellulose or a mixture of microcrystalline cellulose and sodium carboxymethyl cellulose) may also be used for the pellets. Glidants applied during pelletization include e.g. colloidal silicon dioxide, magnesium stearate, talc and starch.

In the conventional pelletization methods the ratio of the product fraction is about 60% by weight. That is why every technical solution resulting in an increase in the product fraction in addition to the maintenance of other important characteristics of the pellets - such as a nearly spherical shape and a suitable quality of the surface - is of high importance.

Several methods aiming at increasing the product fraction have been published. According to European patent specification No. 288,732 during the extrusion-spheroidization process organic acids (such as citric, tartaric, maleic, fumaric, succinic acid) applied in the composition in an amount of 5 to 50% by weight improve the plasticity of the wet mass and thus enable the preparation of harder pellets with particles of a narrow size range. At the same time this method cannot be used for the formulation of acid-sensitive active ingredients.

According to a publication of G.A. Hileman et al. (Drug Dev. and Ind. Pharmacy, 19(4), 483-491, 1993) in case of the preparation of pellets with high active agent content the application of microcrystalline cellulose containing a slight amount of sodium carboxymethyl cellulose promote the formation of high-quality pellets.

In the process disclosed in International patent application No. WO 94/08567 0.03% by weight of polysorbate is applied in order to increase the ratio of product fraction.

According to the process disclosed in Hungarian patent specification No. 198,396 a coating powder containing 5 to 98% by weight of low-substituted hydroxypropyl cellulose (containing 4 to 20% of hydroxypropyl group) is applied during the pelletization.

On the basis of the above facts it can be established that pelletization is a complicated pharmaceutical operation requiring a special equipment and intensive labour.

For the persons skilled in the manufacture of pharmaceutical compositions it is known that by using the excipients known from the prior art it is particularly difficult - in certain cases impossible - to produce pellets in suitable quality (which are approximately spherical in shape) and in high yield (with a high ratio of the product fraction), especially in case of active ingredients soluble or readily soluble in water.

Venlafaxine hydrochloride belongs to the group of the active ingredients readily soluble in water (0.57 g/ml).

According to Hungarian patent specification No. 196,677 pellets cannot be prepared from methoprolol salts readily soluble in water, that is from salts having a water-solubility higher than 600 mg/ml. What is more, even in case of methoprolol salts having a water solubility lower than 600 mg/ml the application of a special technology is necessary. During this technology the active ingredient is applied onto water-insoluble quartz or glass granules from a solution of methoprolol fumarate or succinate in ethanol-dichloromethane by a layering process. This technology requires an expensive equipment from the viewpoint of solvent recovery and regeneration, its productivity is poor, and it has unfavourable characteristics considering environmental, health and fire protection.

Hungarian patent application No. P9700589 suggests that in case of venlafaxine hydrochloride having a solubility higher than 500 mg/ml pelletization can be performed only by the application of microcrystalline cellulose combined with hydroxypropyl-methyl cellulose.

According to Hungarian patent application No. P0004287 pellets containing venlafaxine can also be prepared without the application of hydroxy-propyl-methyl cellulose if the ratio of the weight of venlafaxine is decreased in the pellets. However, this solution is unfavourable, because in case of identical doses a decrease in the weight ratio of venlafaxine constitutes a higher pellet weight and consequently a medicine of bigger size. Thus it is more difficult to swallow the composition and the manufacture is more expensive.

EP-A-1 331 003 describes encapsulated extended release formulations containing venlafaxine. HCl (in polymorphic forms I or II) in combination with microcrystalline cellulose and hydroxypropylmethylcellulose.

WO 03/082261 A1 describes extended release formulations of venlafaxine. HCl containing sodium carboxymethyl cellulose.

WO 03/041692 A1 describes extended release venlafaxine. HCl formulations where venlafaxine is coated on microcrystalline cellulose which is then coated with a polymeric layer for extended release.

None of EP-A-1 331 003, WO 03/082261 A1 and WO 03/041692 A1 describes or suggests venlafaxine. HC1 pellets comprising NaCl and/or KCl and microcrystalline cellulose.

Data of the technical literature show that in case of the readily water-soluble venlafaxine hydrochloride experts have so far failed to elaborate a composition of the excipients enabling the preparation of pellets having a high active ingredient content and a suitable quality (being approximately spherical in shape) and providing an advantageous product fraction.

The aim of the invention was to elaborate a composition and process which are suitable for the preparation of pellets in up-to-date fluidization and rotogranulation machines providing the preparation of the product fraction (that is the ratio of pellets used for further processing) in a high yield, that is in a yield of at least 75%, preferably more that 80%, and in a suitable quality.

### Summary of the invention .

The invention is based on the surprising recognition that if 10 to 60% by weight of sodium chloride and/or potassium chloride are added to the powder mixture to be pelletized in addition to at most 80% by weight of active agent and 10 to 60% by weight of microcrystalline cellulose, pellets with high active ingredient content and very advantageous in shape (approximately spherical) can be produced from the otherwise unpelletizable active agents as well. Besides, the particle size of the said pellets is highly suitable for pharmaceutical purposes, and the yield of the product fraction exceeds 75%.

### Details of the invention

A subject-matter of the present invention are pellets in nearly spherical shape comprising pharmaceutically active venlafaxine hydrochloride, whereby said pellets contain (related to the total weight) at most 80% by weight of venlafaxine hydrochloride, 10 to 60% by weight of sodium chloride and/or potassium chloride, 10 to 60% by weight of microcrystalline cellulose and optionally other pharmaceutically acceptable excipients and/or pelletization promoting additives.

Advantageously the pellet has a particle size in the range between 0.2 mm and 2 mm, preferably between 0.5 mm and 1.6 mm.

Preferred pellets comprise 0.4 to 60% by weight of active ingredient, 20 to 60% by weight of sodium chloride and/or potassium chloride, 20 to 60% by weight of microcrystalline cellulose, 0.5 to 3.0% by weight of dimethyl polysiloxane and/or 0.1 to 1.0% by weight of xanthane gum and, if desired, 0.1 to 0.6% by weight of colloidal silicon dioxide.

In the pellets preferably the total amount of the potassium chloride and sodium chloride is 20 to 60% by weight.

It is also preferred that the weight ratio of the sodium chloride:potassium chloride is about 20:1.

According to an embodiment of the invention the pellets comprise venlafaxine hydrochloride in the polymorphic form I.

According to another embodiment of the invention the pellets comprise venlafaxine hydrochloride in the polymorphic form II.

According to a still further embodiment of the invention the pellets comprise venlafaxine hydrochloride in the polymorphic form III. The pellets optionally are supplied with a coating to modify the drug release and have a particle size between 0.8 mm and 1.6 mm, which comprise 35 to 45% by weight of venlafaxine hydrochloride in admixture with 25 to 35% by weight of microcrystalline cellulose, 1.0 to 1.6% by weight of potassium chloride, 23.8 to 30% by weight of sodium chloride, 0.5 to 1.6% by weight of dimethyl polysiloxane and/or 0.1 to 0.2% by weight of xanthane gum and 0.4 to 0.6% by weight of colloidal silicon dioxide.

According to a further aspect of the invention there are provided for solid pharmaceutical compositions comprising as active ingredient venlafaxine hydrochloride formulated with the aid of pellets according to the invention.

According to a still further aspect of the present invention there is provided for a process for the preparation of the nearly spherical pellets containing the pharmaceutical ingredient venlafaxine, which comprises admixing at most 80% of the active ingredient with 10 to 60% by weight of microcrystalline cellulose, 10 to 60% by weight of sodium chloride and/or potassium chloride and optionally other, pharmaceutically acceptable excipients and/or additives promoting the pelletization, converting the thus-obtained mixture into pellets by high-shear mixing, rotofluidization or extrusion-spheroidization method, preferably by rotofluidization by spraying water or preferably an aqueous dimethyl polysiloxane emulsion and/or an aqueous solution of xanthane gum, drying the pellets, separating the desired product fraction and optionally coating the pellets with a layer to modify the drug release.

In order to improve efficiency of the pelletization dimethyl polysiloxane is emulged in the pelletizing liquid. In this way the particle size of the thus-obtained pellets can be controlled much better, the size or the pellets is more uniform and the product fraction may exceed even 90%. Similar results can be achieved when xanthane gum is dissolved in the pelletezing liquid, or when the pelletizing liquid contains both dimethyl polysiloxane and xanthane gum. The exact mechanism of the effect of sodium chloride and/or potassium chloride, dimethyl polysiloxane and xanthane gum is not known at the moment, but it can be supposed that the formation of a more uniform particle size may be the result of a decrease in the solubility of the active ingredient upon the effect of the ionic filler materials, an increase in the plasticity of the powder mixture to be pelletized and some advantageous changes of the cohesion between the particles.

The pharmaceutical compositions containing pelletized active ingredient are usually prepared in dosage unit forms. During the pelletization process the concentration of the active ingredient is determined by the single dose of the active agent finished in a dose unit so that the weight of the pellets containing a single dose of the active ingredient should vary between 50 to 1500 mg, preferably between 100 to 700 mg.

In the knowledge of the weight of the pellets containing a single dose of the active ingredient the sodium chloride and/or potassium chloride and microcrystalline cellulose content of the pellet can be determined. The premix used for the preparation of pellets according to the invention possessing advantageous properties should contain a total amount of at least 10% by weight of both excipients. If the active ingredient content of the pellets is lower than 80% by weight as a consequence of a lower dose, the sodium chloride and/or potassium chloride and microcrystalline cellulose content may be increased. The two types of excipients can be used in nearly identical amounts.

As to the quality of the microcrystalline cellulose used for the preparation of pellets according to the invention, there are no restrictions. Various types of microcrystalline cellulose of different particle size and of different density may freely be applied. Even the application of microcrystalline cellulose with a low moisture content is not required, since during pelletization the substance mixture is moistened with water or contacted with an aqueous solution or dispersion. In addition to the so-called pure or monocomponent microcrystalline cellulose varieties mixtures of various types of microcrystalline cellulose formed with colloidal silicon dioxide can also be used, which contain about 98% by weight of microcrystalline cellulose and about 2% by weight of colloidal silicon dioxide.

From the viewpoint of pelletization both sodium chloride and potassium chloride can be used to advantage, but due to their different physiological effects it is preferable to chose their concentrations on the basis of the extracellular physiological concentration. This constitutes a weight ratio of sodium chloride to potassium chloride of about 20:1. Thus, choosing e.g. 10% by weight as sodium chloride/potassium chloride concentration, preferably 9.5% by weight of sodium chloride and 0.5% by weight of potassium chloride can be applied.

Furthermore, by applying excipients in the compositions of the pellets according to the invention, the quality of the pellets and the quantity of the amount of pellets falling into the preferable particle size region can be improved. For this purpose xanthane gum or dimethyl polysiloxane may be used. These auxiliary agents are dissolved or disperged either individually or together in water used for the pelletization, and added to the premix to be pelletized in the beginning of the pelletization procedure.

Xanthane gum used in the compositions according to the invention is a natural polysaccharide produced by the microorganism *Xantomonas campestris.* The skeleton of xanthane gum, such as that of cellulose, consists of 1,4-glucopyranose units, while the side-chains contain mannose, acetate and glucuronic acid. The average molecular weight is a few million Dalton.

In the pharmaceutical industry xanthane gum is mostly applied for increasing the stability of suspensions used for coating of medicines or films (e.g. Hungarian patent specification No. 202120) or as a viscosity-increasing agent (the United States Pharmacopoeia, ed. 26., 2003, United States Pharmacopoeial Convention, Inc, Rockwille, USA) or as a matrix building material for extended release tablets. When used for pelletization, xanthane gum increases the stability of the pellets. This advantageous effect of this substance has not so far been mentioned in the literature and could not be aforeseen. Xanthane gum can be used in the pelletization liquid in an amount of not exceeding 2% by weight related to the final weight of the pellet.

Dimethyl polysiloxane used in the compositions according to the invention is a liquid substance, a completely methylated polymeric siloxane having a viscosity value between 100 and 1000 centi-stokes. The aqueous dimethyl polysiloxane dispersions are commercially available products, which are used for surface treatments, as skin softening additives for pharmaceutical and cosmetic compositions, as antifriction agents for tabletting or as antiadhesives for film-coating (Hungarian patent specification No. 190,693). The United States Pharmacopoeia (USP 26) classifies dimethyl polysiloxane into the group of antifoam additives. A surprising - and so far fully unknown - property of dimethyl polysiloxane is that in the course of pelletization, when emulged in an amount of not exceeding 5% by weight into water or an aqueous colloidal solution of xanthane gum used for the pelletization, it improves the spherical shape of the pellets and augments the ratio of the product fraction.

The pellets according to the invention can be prepared by build-up (high-shear or roto-fluidization) or by extrusion-spheroidization methods known from the literature. In the course of the pelletization process venlafaxine hydrochloride is mixed with sodium chloride and/or potassium chloride and microcrystalline cellulose The mixture is homogenized and the thus-obtained powdered premix is moistened with water or with an aqueous solution of the binder(s) and/or other, pharmaceutically acceptable excipients. The mixture is converted into pellets of the desired particle size by using an appropriate equipment (extruder-spheroidizator-drier, high-shear granulator-drier, rotofluidization apparatus). Then the product fraction having the desired particle size is isolated by size, e.g. by sieving. The pellet fraction having an unsuitable particle size is transferred back to the pelletization process after grinding. The advantageous effect of sodium chloride and potassium chloride on the pelletization manifests itself in the simplification of the manufacturing technology, increase in its reliability and the advantageous formation of the product fraction.

Another advantage of the application of the above-mentioned alkali salts is that their price is low even in case of a pharmaceutical quality, so their application results in a reduction of the manufacturing costs.

The pellets containing venlafaxine hydrochloride according to the invention are particularly suitable for further coating operations. In the course of coating water-soluble (e.g. hydroxypropyl-methyl cellulose, polyvinyl alcohol), water-insoluble (e.g. ethyl cellulose, ethyl acrylate - methyl methacrylate copolymer, polyvinyl acetate) or film-forming substances having a solubility depending on the pH of the aqueous medium (e.g. cellulose acetate phtalate, hydroxypropyl-methyl cellulose acetate succinate, ethyl-acrylate-methacrylic acid copolymer) can equally be used. Coating can be carried out with an aqueous or organic solution or an aqueous dispersion of the film-forming substances. Depending on the solubility characteristics of the film coating immediate release (water-soluble coating), extended release (water-insoluble coating) or delayed release (the solubility of the coating depends on the pH of the releasing medium) compositions can be prepared. The quality and quantity of the coating substance are to be determined on the basis of the dissolution profile, that is the desired dependence of the release on time.

The pellets containing venlafaxine hydrochloride according to the invention and preparation thereof are illustrated by the following examples.

### Example 1

### Preparation of pellets containing venlafaxine hydrochloride

450 g of venlafaxine hydrochloride, 396 g of microcrystalline cellulose, 6 g of colloidal silicon dioxide, 342 g of sodium chloride and 18 g of potassium chloride were mixed in an acid-resistant vessel. The mixture was transferred to a fluidization rotogranulator (Glatt GPCG1), and a mixture of 20 g of 35% dimethyl polysiloxane emulsion and 1000 ml of ion-exchanged water was sprayed onto it. Spraying speed of the pelletizing liquid was set at 50 ml/min, pressure of the spraying air was 2.5 bar. The speed of the rotor was set at 450 rev/min in the first 15 minutes of the pelletization and later kept at 600 rev/min. Speed by volume of the fluidization air was kept at 60 m³/hour in the first 15 minutes of the pelletization and later at 90 m³/hour. The temperature of the fluidization air was set at 25 °C in the first part of the pelletization and to 40 °C for the drying procedure.

The dried pellets were passed through sieves having hole widths of 0,8 mm and 1,6 mm, respectively and divided into 3 fractions. The product fraction, that is the amount of the granules in the range between 0.8 mm and 1.6 mm, amounts to 96% by weight.

For the preparation of a sustained release composition a fluidization apparatus of Glatt GPCG1 type was equipped with a bottom spraying Wurster insert, and 500 g of the product fraction were fed into the container. As coating liquid 200.0 g of an aqueous polymeric dispersion containing 30% of polyvinyl acetate (trademark name: Kollicoat SR) were applied. To the said dispersion 8.4 g of propylene glycol dissolved in 240 g of water were added. Coating was carried out under the following conditions:
- temperature of the fluidization air: 60 °C
- rate of feed: 8 ml/min
- pressure of spraying: 2 bar

### Example 2

### Preparation of pellets containing venlafaxine hydrochloride

450 g of venlafaxine hydrochloride, 396 g of microcrystalline cellulose, 6 g of colloidal silicon dioxide, 342 g of sodium chloride and 18 g of potassium chloride were mixed in an acid-proof vessel. The mixture was transferred to a fluidization rotogranulator (Glatt GPCG1), and a. solution of 2 g of xanthane gum in 300 ml of ion-exchanged water and further 1080 ml of ion-exchanged water were sprayed onto it. Spraying speed of the pelletizing liquid was set at 50 ml/min, pressure of spraying air was 2.5 bar. The speed of the rotor was set at 450 rev/min in the first 15 minutes of the pelletization and later kept at 600 rev/min. Speed by volume of the fluidization air was kept at 60 m³/hour in the first 15 minutes of the pelletization and later at 90 m³/hour. The temperature of the fluidization air was set at 25 °C in the first part of the pelletization and to 40°C for the drying procedure.

The dried pellets were passed through sieves having hole widths of 1.6 mm and 0.8 mm, respectively and divided into three fractions. The product fraction, that is the amount of the pellets in the range between 0.8 mm and 1.6 mm, amounts to 90% by weight.

### Example 3

### Preparation of pellets containing venlafaxine hydrochloride

450 g of venlafaxine hydrochloride, 396 g of microcrystalline cellulose, 6 g of colloidal silicon dioxide, 342 g of sodium chloride and 18 g of potassium chloride were mixed in an acid-proof vessel. The mixture was transferred to a fluidization rotogranulator (Glatt GPCG1), and a mixture of a solution of 2 g of xanthane gum in 250 ml of ion-exchanged water and 50 g of 35% dimethyl polysiloxane and a mixture of further 60 g of 35% dimethyl polysiloxane and 1000 ml of ion-exchanged water were sprayed onto it. Spraying speed of the pelletizing liquid was set at 50 ml/min, pressure of spraying air was 2.5 bar. The speed of the rotor was set at 450 rev/min in the first 15 minutes of the pelletization and later kept at 600 rev/min. Speed by volume of the fluidization air was kept at 60 m³/hour in the first 15 minutes of the pelletization and later at 90 m³/hour. The temperature of the fluidization air was set at 25 °C in the first part of the pelletization and to 40 °C for the drying procedure.

The dried pellets were passed through sieves, having hole widths of 1.6 mm and 0.8 mm, respectively and divided into 3 fractions. The product fraction, that is the amount of the granules in the range between 0.8 mm and 1.6 mm, amounts to 88.3% by weight.

For the preparation of sustained-release compositions a fluidization apparatus of Glatt GPCG1 type is equipped with a bottom spraying Wurster insert, and 500 g of the above product fraction were fed into the container. As coating liquid a solution of 60.0 g of ethylcellulose (viscosity: 10 centipoise) and 6.0 g of copovidone (vinylpyrrolidone acetate (60/40) copolymer) in 550.0 g of 96% by volume ethanol were used, in which 18.0 g of talc had been suspended as antiadhesive. Coating was carried out under the following conditions:
- temperature of the fluidization air: 40 °C
- rate of feed: 8 ml/min
- pressure of spraying: 2 bar

### Example 4

### Preparation of pellets containing venlafaxine hydrochloride

450 g of venlafaxine hydrochloride, 396 g of microcrystalline cellulose, 6 g of colloidal silicon dioxide, 342 g of sodium chloride and 18 g of potassium chloride were mixed in an acid-proof vessel. The mixture was transferred to a fluidization rotogranulator (Glatt GPCG1), and 1500 ml of ion-exchanged water were sprayed onto it. Spraying speed of the pelletizing liquid was set at 50 ml/min, pressure of spraying air was 2.5 bar. The speed of the rotor was set at 450 rev/min in the first 15 minutes of the pelletization and later kept at 600 rev/min. Speed by volume of the fluidization air was kept at 60 m³/hour in the first 15 minutes of the pelletization and later at 90 m³/hour. The temperature of the fluidization air was set at 25 °C in the first part of the pelletization and at 40 °C for the drying procedure.

The dried pellets were passed through sieves having hole widths of 1.6 mm and 0.8 mm, respectively, and divided into 3 fractions. The product fraction, that is the amount of the granules in the range between 0.8 mm and 1.6 mm, amounts to 75.3% by weight.

### Example 5

### Preparation of pellets containing venlafaxine hydrochloride

11.0 kg of venlafaxine hydrochloride, 7.26 kg of microcrystalline cellulose, 0.13 kg of colloidal silicon dioxide, 5.97 kg of sodium chloride and 0.32 kg of potassium chloride were homogenized in a high-shear granulator of Diosna V-100 type. The homogenizate was moistened with a mixture of a solution of 0.04 kg of xanthane gum in 6.5 kg of ion-exchanged water and 1.0 kg of 39% dimethyl polysiloxane. The wet substance was sieved with the aid of an oscillation apparatus of Diaf GS-140 type using a sieve having hole widths of 1,0 mm. The sieved substance was transferred to a fluidization rotogranulator of Glatt GPCG15 type, about 20 kg of ion-exchanged water were sprayed onto it and converted into pellets having a particle size mostly in the range between 0.8 mm and 1.6 mm. Spraying speed of the pelletizing liquid was set at 250 ml/min, pressure of spraying air was 3.5 bar. The speed of the rotor was kept at 300 rev/min during the pelletization and at 500 rev/min during drying of the pellets. Speed by volume of the fluidization air was varied between 350 and 750 m³/hour depending on the motion of the material. The temperature of the fluidization air was kept at 25 °C during the pelletization and at 40 °C in the course of the drying.

For the preparation of sustained-release compositions a fluidization apparatus of Glatt GPCG1 type was equipped with a bottom spraying Wurster insert, and 20 kg of the above product fraction were fed into the container. As coating liquid a solution of 3.30 kg g of ethylcellulose (viscosity: 10 centipoise) and 0.33 kg of copovidone (vinylpyrrolidone/vinylacetate (60/40) copolymer) in 30 kg of 96% by volume ethanol was used, in which as antiadhesive 0.99 kg of talc and as colouring agent 0.02 kg of iron oxide pigment had been suspended. Coating was carried out under the following conditions:
- temperature of the fluidization air: 60 °C
- rate of feed: 90 ml/min
- pressure of spraying: 3.5 bar

## Claims

1. Pellets in nearly spherical shape comprising pharmaceutically active venlafaxine hydrochloride, whereby said pellets contain (related to the total weight) at most 80% by weight of venlafaxine hydrochloride, 10 to 60% by weight of sodium chloride and/or potassium chloride, 10 to 60% by weight of microcrystalline cellulose and optionally other pharmaceutically acceptable excipients and/or pelletization promoting additives.

2. Pellets according to claim 1, wherein the pellet has a particle size in the range between 0.2 mm and 2 mm, preferably between 0.5 mm and 1.6 mm.

3. Pellets according to claim 2 comprising 0.4 to 60% by weight of active ingredient, 20 to 60% by weight of sodium chloride and/or potassium chloride, 20 to 60% by weight of microcrystalline cellulose, 0.5 to 3.0% by weight of dimethyl polysiloxane and/or 0.1 to 1.0% by weight of xanthane gum and, if desired, 0.1 to 0.6% by weight of colloidal silicon dioxide.

4. Pellets according to claim 3, wherein the total amount of the potassium chloride and sodium chloride is 20 to 60% by weight.

5. Pellets according to claim 4, wherein the weight ratio of the sodium chloride:potassium chloride is about 20:1.

6. Pellets according to any of claims 1 to 5 comprising venlafaxine hydrochloride in the polymorphic form I.

7. Pellets according to any of claims 1 to 5 comprising venlafaxine hydrochloride in the polymorphic form II.

8. Pellets according to any of claims 1 to 5 comprising venlafaxine hydrochloride in the polymorphic form III.

9. Pellets according to claim 8 optionally supplied with a coating to modify the drug release and having a particle size between 0.8 mm and 1.6 mm, which comprise 35 to 45% by weight of venlafaxine hydrochloride in admixture with 25 to 35% by weight of microcrystalline cellulose, 1.0 to 1.6% by weight of potassium chloride, 23.8 to 30% by weight of sodium chloride, 0.5 to 1.6% by weight of dimethyl polysiloxane and/or 0.1 to 0.2% by weight of xanthane gum and 0.4 to 0.6% by weight of colloidal silicon dioxide.

10. Solid pharmaceutical compositions comprising as active ingredient venlafaxine hydrochloride formulated with the aid of pellets as claimed in any of claims 1 to 9.

11. A process for the preparation of pellets according to any of claims 1 to 9, which comprises admixing at most 80% of the active ingredient with 10 to 60% by weight of microcrystalline cellulose, 10 to 60% by weight of sodium chloride and/or potassium chloride and optionally other, pharmaceutically acceptable excipients and/or pelletization promoting additives, converting the thus-obtained mixture into pellets by high-shear blending, rotofluidization or extrusion-spheroidization method, preferably by rotofluidization by spraying water or preferably an aqueous dimethyl polysiloxane emulsion and/or an aqueous solution of xanthane gum, drying the pellets, separating the desired product fraction and optionally coating the pellets with a layer to modify the drug release.

## Patentansprüche

1. Pellets in nahezu kugelförmiger Gestalt, umfassend pharmazeutisch aktives Venlafaxinhydrochlorid, wobei die Pellets (bezogen auf das Gesamtgewicht) höchstens 80 Gew.-% Venlafaxinhydrochlorid, 10 bis 60 Gew.-% Natriumchlorid und/oder Kaliumchlorid, 10 bis 60 Gew.-% mikrokristalline Zellulose und optional weitere pharmazeutisch zulässige Hilfsstoffe und/oder pelletierungsfördernde Zusatzstoffe enthalten.

2. Pellets nach Anspruch 1, wobei das Pellet eine Partikelgröße im Bereich von 0,2 mm bis 2 mm, vorzugsweise von 0,5 mm bis 1,6 mm aufweist.

3. Pellets nach Anspruch 2, umfassend 0,4 bis 60 Gew.-% aktiven Bestandteil, 20 bis 60 Gew.-% Natriumchlorid und/oder Kaliumchlorid, 20 bis 60 Gew.-% mikrokristalline Zellulose, 0,5 bis 3,0 Gew.-% Dimethylpolysiloxan und/oder 0,1 bis 1,0 Gew.% Xanthangummi und, falls gewünscht, 0,1 bis 0,6 Gew.-% kolloidales Siliciumdioxid.

4. Pellets nach Anspruch 3, wobei die Gesamtmenge des Kaliumchlorids und Natriumchlorids 20 bis 60 Gew.% beträgt.

5. Pellets nach Anspruch 4, wobei das Gewichtsverhältnis von Natriumchlorid zu Kaliumchlorid etwa 20:1 beträgt.

6. Pellets nach einem der Ansprüche 1 bis 5, umfassend Venlafaxinhydrochlorid in der polymorphen Form I.

7. Pellets nach einem der Ansprüche 1 bis 5, umfassend Venlafaxinhydrochlorid in der polymorphen Form II.

8. Pellets nach einem der Ansprüche 1 bis 5, umfassend Venlafaxinhydrochlorid in der polymorphen Form III.

9. Pellets nach Anspruch 8, die optional mit einer Schicht versehen sind, um die Arzneistofffreisetzung zu modifizieren, und eine Partikelgröße von 0,8 mm bis 1,6 mm aufweisen, wobei sie 35 bis 45 Gew.-% Venlafaxinhydrochlorid unter Beimengung von 25 bis 35 Gew.-% mikrokristalline Zellulose, 1,0 bis 1,6 Gew.-% Kaliumchlorid, 23,8 bis 30 Gew.-% Natriumchlorid, 0,5 bis 1,6 Gew.-% Dimethylpolysiloxan und/oder 0,1 bis 0,2 Gew.% Xanthangummi und 0,4 bis 0,6 Gew.-% kolloidales Siliciumdioxid umfassen.

10. Feste pharmazeutische Mischungen, umfassend Venlafaxinhydrochlorid, formuliert mithilfe der Pellets nach einem der Ansprüche 1 bis 9, als aktiven Bestandteil.

11. Verfahren zum Herstellen von Pellets nach einem der Ansprüche 1 bis 9, umfassend die Schritte: Beimengen von höchstens 80% des aktiven Bestandteils mit 10 bis 60 Gew.-% mikrokristalline Zellulose, 10 bis 60 Gew.-% Natriumchlorid und/oder Kaliumchlorid, und optional von weiteren pharmazeutisch zulässigen Hilfsstoffe und/oder pelletierungsfördemden Zusatzstoffen, welche die so erhaltene Mischung durch ein High-Shear-Misch-, Roto-Fluidisierungs- oder Extrusions-Kugelbildungsverfahren, vorzugsweise durch Roto-Fluidisierung durch Versprühen von Wasser oder vorzugsweise einer wässrigen Dimethylpolysiloxan-Emulsion und/oder einer wässrigen Lösung aus Xanthangummi in Pellets umwandelt, Trocknen der Pellets, Trennen der gewünschten Produktfraktion und optional Beschichten der Pellets mit einer Schicht, um die Arzneistofffreisetzung zu modifizieren.

## Revendications

1. Pellets de forme presque sphériques comprennent de venlafaxine chlorhydrate, substance pharmaceutiquement active, où les-dits pellets contiennent (en rapport avec le poids total) au plus 80% en poids de venlafaxine chlorhydrate, 10% à 60% en poids de chlorure de sodium et/ou de chlorure de potassium, 10 à 60% en poids de cellulose microcrystalline et optionnellement d'autres excipients pharmaceutiquement acceptables et/ou des additifs favorisant la pelletisation.

2. Pellets selon la revendication 1, où le pellet a une dimension se situant entre 0,2 mm et 2 mm, de préférence entre 0,5 mm et 1,6 mm.

3. Pellets selon la revendication 2 comprennent 0,4 à 60% en poids d'ingrédients actifs, de 20% à 60% en poids de chlorure de sodium et/ou de chlorure de potassium, 20% à 60% en poids de cellulose microcrystalline, de 0,5% à 3,0% en poids de diméthyle-polysiloxane et /ou 0,1% à 1,0% en poids de gomme de xanthane, et si l'on souhaite de 0,1% à 0,6% en poids de dioxyde de silicone colloïdal.

4. Pellets selon la revendication 3, où la somme totale de chlorure de potassium et de chlorure de sodium est de 20% à 60% en poids.

5. Pellets selon la revendication 4 où la proportion de chlorure de sodium : chlorure de potassium est environ de 20 : 1.

6. Pellets selon l'une des revendications 1 à 5 comprenant de venlafaxine chlorhydrate de forme polymorphe I.

7. Pellets selon l'une des revendications 1 à 5 comprenant de venlafaxine chlorhydrate de forme polymorphe II.

8. Pellets selon l'une des revendications 1 à 5 comprenant de venlafaxine chlorhydrate de forme polymorphe III.

9. Pellets selon la revendication 8 sont fournis avec un enrobage facultatif destiné à modifier la libération du médicament et ont une taille de particule qui se situe entre 0,8 mm et 1,6 mm, ce qui constitue 35 à 45% en poids de venlafaxine chlorhydrate en mélange avec 25 à 35% en poids de cellulose microcrystalline, 1,0 à 1,6% en poids de chlorure de potassium, 23,8 à 30% en poids de chlorure de sodium, de 0,5% à 1,6% en poids de diméthyle-polysiloxane et /ou 0,1% à 0,2% en poids de gomme de xanthane, et de 0,4% à 0,6% en poids de dioxyde de silicone colloïdal.

10. Compositions pharmaceutiques comprennent comme ingrédient actif de venlafaxine chlorhydrate formulée à l'aide de granules comme il est mentionné dans l'une des revendications de 1 à 9.

11. Procédé pour la préparation de granules selon l'une des revendications de 1 à 9, comprenant en mélange 80% d'ingrédient actif au plus avec 10 à 60% en poids de cellulose microcrystalline, 10 à 60% en poids de chlorure de sodium et/ou de chlorure de potassium et éventuellement d'autres excipients pharmaceutiquement acceptables et/ou des additifs favorisant pelletisation, transformant le mélange ainsi obtenu en granules par différentes méthodes : soit par un mélange sous cisaillement, soit par roto-fluidisation ou par extrusion-sphéroidisation, de préférence par roto-fluidisation en vaporisant de l'eau, ou mieux une émulsion de diméthyle-polysiloxane aqueuse et/ou une solution aqueuse de gomme de xanthane, en desséchant les pellets, en séparant les fractions de produit souhaité et en enrobant éventuellement les pellets avec une pellicule qui modifie la libération du médicament.
